# EUROPÄISCHE PATENTSCHRIFT

(11) **EP 2 216 648 B1**
(45) Veröffentlichungstag und Bekanntmachung des Hinweises auf die Patenterteilung: **10.04.2013**
(21) Anmeldenummer: 09001639.5
(22) Anmeldetag: 05.02.2009
(51) Int. Cl.: G01N 31/22, G01N 21/78

(54) **Reinigungsindikator und Prüfkörper zur Prüfung von Reinigungsprozessen**
Cleaning indicator and test body for testing cleaning processes
Indicateur de nettoyage et corps de contrôle associé pour contrôle de processus de nettoyage

(43) Veröffentlichungstag der Anmeldung: 11.08.2010
(73) Patentinhaber: Kaiser, Danja, 61479 Glashütten (DE)
(72) Erfinder: Kaiser, Danja, 61479 Glashütten (DE)
(74) Vertreter: Tergau & Walkenhorst

(56) Entgegenhaltungen:
- DE-A1- 10 065 941
- DE-A1-102006 029 485
- JP-A- 6 034 621
- US-A1- 2003 041 655

## Beschreibung

Die Erfindung bezieht sich auf einen Reinigungsindikator zur Prüfung von Reinigungsprozessen. Sie bezieht sich weiterhin auf einen zugehörigen Prüfkörper und ein Verfahren zur Prüfung von Reinigungsprozessen.

Reinigungsprozesse, insbesondere in Labors und Krankenhäusern durchgeführte maschinelle Reinigungsprozesse, dienen der Entfernung von zum Teil sehr hartnäckigem Schmutz und Ablagerungen von beispielsweise medizinischen Instrumenten oder Laborgeräten. Die Reinigung geschieht üblicherweise in Reinigungsautomaten, die in ihrer Funktionsweise den haushaltsüblichen Spülmaschinen ähneln. Diese Geräte werden als Reinigungs-Desinfektions-Geräte (RGD) bezeichnet. Sie führen verschiedene Spülgänge, ggf. in Kombination mit Desinfektionsschritten, durch (Vorspülen, Reinigen, Desinfizieren und Klarspülen). Wiederverwendbare medizinische Geräte und Laborgeräte, insbesondere chirurgische Instrumente, müssen zusätzlich vor ihrer Verwendung sterilisiert werden. Bei aseptischen Anwendungen ist die Verwendung von sterilen Werkzeugen, Instrumenten oder Materialien zwingend erforderlich. Dazu folgt dem Reinigungsprozess ein Sterilisationsprozess.

Zur Beurteilung, ob ein Reinigungsprozess erfolgreich war, können Indikatoren, die beispielsweise sogenannte Testanschmutzungen umfassen, verwendet werden. Gewöhnlich umfassen diese Indikatoren Schichten aus einem geeigneten Material, das die Anschmutzung der zu reinigenden Beladung simuliert, und sind auf einem wasserfesten Trägermaterial aufgebracht. Diese Schichten sind im Regelfalle farbig. Je nach Bedarf werden für diese Beschichtungen geeignete Materialien verwendet, z.B. Blutbestandteile, Eiweiß, Polysacharide, Polyzucker, Milchproteine. Eine synthetische Testanschmutzung ist beispielsweise aus der EP 0 886 778 B1 bekannt. Verfahren und Vorrichtung zur Bestimmung der Reinigungswirkung von waschvorgängen sind beispielsweise aus der DE 10 2006 029 485 A1 und aus der DE 10065941 A1 bekannt.

Die unterschiedlichen Anschmutzungen stellen verschiedene Anforderungen an die Reinigungswirkung bzw. Reinigungsleistung des Reinigungsprozesses. Damit ist die Effizienz des Reinigungsprozesses von der Art der Verschmutzung abhängig. Die Reinigungswirkung ist in diesem Zusammenhang als integrale Größe gegeben, die sich aus einem Zusammenspiel von z. B. Dauer, Temperatur, Druck, Sprühverhältnissen, Reinigungschemikalien und anderen Reinigungsparametern ergibt.

Bei Verwendung derartiger Indikatoren kann nach dem Reinigungsprozess durch eine visuelle Prüfung des Indikatorträgers festgestellt werden, ob die Indikatorsubstanz vollständig abgewaschen wurde. Die derzeitig auf dem Markt befindlichen Reinigungsindikatoren erlauben keine genaueren bzw. graduelle Angaben über die Bedingungen während des Reinigungsvorgangs und den erzielten Reinigungserfolg, da bisher jegliche Normvorgaben fehlen.

Die Normreihe EN ISO 15883 beschäftigt sich ganz allgemein mit den Themen Reinigung und Desinfektion. Allerdings trifft sie keine Aussagen zu konkreten Prüfmethoden. In der Technischen Information EN ISO 15883-5 sind ca. 30 unterschiedliche Prüfanschmutzungen aufgelistet. In diesem Zusammenhang wird aber keine Aussage über den Schwierigkeitsgrad der Entfernung dieser Beschichtungen während eines Reinigungsprozesses getroffen.

Der Erfindung liegt daher die Aufgabe zugrunde, einen Reinigungsindikator anzugeben, der eine differenzierte und reproduzierbare Prüfung von Reinigungsprozessen erlaubt. Weiterhin sollen dazu besonders geeignete Prüfkörper angegeben werden.

Bezüglich des Reinigungsindikators wird diese Aufgabe erfindungsgemäß gelöst durch die Merkmale des Anspruchs 1.

Vorteilhafte Ausgestaltungen der Erfindung sind Gegenstand der Unteransprüche.

Die Erfindung geht von der Überlegung aus, dass sich Reinigungsprozesse durch ihre Reinigungsleistung charakterisieren lassen. Die Reinigungsleistung sollte dabei den Eigenschaften des zu entfernenden Schmutzes und den jeweiligen Anforderungen an die Gründlichkeit der Reinigung angepasst werden. Nur so kann auf der einen Seite sichergestellt werden, dass der Schmutz auch wirklich entfernt wird, und auf der anderen Seite bei geringeren Erfordernissen bezüglich der Reinigungsleistung Material, Energie und Zeit effektiv eingespart werden. Dabei ist zu beachten, dass in Reinigungsprozessen, wie sie typischerweise in Labors und Krankenhäusern vorkommen, gewöhnlich unterschiedliche Verschmutzungen - mit unterschiedlichen Anforderungen an die Reinigungsleistung - simultan vorkommen und entfernt werden müssen.

Um eine gezielte Anpassung der Reinigungsleistung an die tatsächlichen Erfordernisse bewerkstelligen zu können bzw. nach Beendigung des Reinigungsprozesses dessen Erfolg prüfen zu können, bedarf es daher einer wirksamen Prüfmethode, die den erzielten Reinigungserfolg nicht nur qualitativ anzeigt, sondern die ihn darüber hinaus in möglichst einfacher Weise quantifizierbar macht. Eine derartige Quantifizierung sollte nach Möglichkeit reproduzierbar und frei von willkürlichen Bewertungsspielräumen sein. Wie sich nunmehr herausgestellt hat, lassen sich diese Anforderungen mit den bislang bekannten Reinigungsindikatoren nur unzureichend erfüllen. Aufgrund einer fehlenden Referenzanschmutzung haben die auf dem Markt befindlichen Indikatoren vollkommen unterschiedliche Eigenschaften und vermitteln deshalb unterschiedliche, nicht vergleichbare Ergebnisse bei der Prüfung der Reinigungsleistung eines Reinigungsprozesses. Eine differenzierte, graduelle Beurteilung der erzielten Reinigungswirkung im Sinne einer kalibrierbaren Referenzmessung ist damit nicht möglich.

Ausgehend von der Erkenntnis, dass ein Reinigungsindikator zur Vermeidung der oben genannten Nachteile für eine graduelle Anzeige und Evaluierung der Reinigungsleistung ausgelegt sein sollte, ist nunmehr vorgesehen, dass der Reinigungsindikator mit mehreren auf einen Träger aufgebrachten Indikatorelementen ausgestattet ist, die simultan und in gleicher Weise einem Reinigungsprozess ausgesetzt werden können, und die gewissermaßen mit unterschiedlicher Empfindlichkeit oder Sensitivität auf die maßgeblichen Reinigungsparameter reagieren bzw. ansprechen. Dabei ändern die Indikatorelemente ihre Eigenschaften bzw. ihre Beschaffenheit in Abhängigkeit von der Reinigungswirkung des Reinigungsprozesses. Indem diese Abhängigkeit, sprich die Sensitivität in Bezug die maßgeblichen Reinigungsparameter bzw. die integrale Reinigungsleistung, bei den einzelnen Indikatorelementen jeweils unterschiedlich ausgelegt ist, was beispielsweise durch eine geeignete Variation physikalischer oder chemischer Strukturparameter oder durch eine geeignete Materialwahl der Indikatorsubstanz realisierbar ist, reagieren die Indikatorelemente in unterschiedlicher Weise und/oder unterschiedlich stark auf ein- und denselben Reinigungsvorgang. Je nachdem, wie viele oder In welchem Maße die Indikatoren in einem Reinigungsprozess ihre Eigenschaften ändern, kann so in der Art einer diskretisierten Bewertung auf die Reinigungsleistung des Prozesses geschlossen werden. Somit wird eine graduelle, quantitativ abgestufte Aussage über die Reinigungsleistung ermöglicht. Auf der Grundlage einer derartigen Quantifizierung können individuelle Reinigungsprozesse beurteilt und deren Leistungsstufe dem Anwendungsfall angepasst werden. Änderungen der Reinigungsleistung eines Prozesses sind damit von Reinigungscharge zu Reinigungscharge erkennbar. Durch Tests mit unterschiedlichen Leistungsstufen lassen sich zudem kalibrierte Referenzdaten gewinnen, die dann später zur Beurteilung bzw. Prüfung von Reinigungsprozessen herangezogen werden können. Die Änderung der Eigenschaften der einzelnen Indikatorelemente führt zu einer jeweils unterschiedlichen Anzeige, die zur Beurteilung der Reinigungswirkung herangezogen werden kann.

Erfindungsgemäß ändern die Indikatorelemente ihre Eigenschaften, sobald die Reinigungswirkung einen vorgegebenen Sollwert erreicht. Dabei sind den einzelnen Indikatorelementen jeweils unterschiedliche Sollwerte zugeordnet. In vielen Fällen ist es von Interesse, ob die Reinigungswirkung einen gewissen Sollwert und damit auch eine gewisse Schwelle erreicht bzw. über- oder unterschritten hat. In dieser Ausführungsform ist somit gewissermaßen eine quantisierte Erfassung der Reinigungswirkung möglich.

Dabei können die Indikatorelemente in Bezug auf ihre Sollwerte geordnet und beispielsweise in auf- oder absteigender Reihenfolge nebeneinander oder übereinander angeordnet sein. Darüberhinaus sind je nach Bedarfsfall auch andere Anordnungen vorzuziehen. Eine geeignete und dem Bedarfsfall angepasste Anordnung der Indikatorelemente ermöglicht eine schnelle und effektive Evaluierung bzw. Beurteilung des Reinigungsprozesses durch Inaugenscheinnahme der Indikatorelemente.

Als Reinigungsmedium können Flüssigkeiten wie Wasser oder Reinigungslösungen, die eine geeignete chemische Zusammensetzung haben, Verwendung finden. Je nach Bedarf kann aber auch eine andere Wahl getroffen werden, z.B. bei einer Dampfreinigung, bei der sich das Reinigungsmedium in dampfförmigen Zustand befindet.

Vorzugsweise erfolgt die Änderung der Indikatorelemente in irreversibler Weise. So kann nach Beendigung des Reinigungsvorgangs zuverlässig nachgewiesen werden, dass zumindest zeitweise eine vorgegebene Reinigungswirkung bzw. Reinigungsleistung, auf die mindestens eines der Indikatorelemente reagiert hat, während des Reinigungsprozesses erreicht wurde. Sind die Indikatorelemente beispielsweise derart ausgelegt, dass sie sich bei Erreichen einer gewissen Reinigungsleistung irreversibel ändern, und hat sich während des Reinigungsprozesses mindestens ein Indikatorelement nicht geändert, kann auf die maximale Reinigungswirkung des Reinigungsprozesses geschlossen werden.

Die Änderung der Eigenschaften der Indikatorelemente kann eine Farbänderung umfassen. Ist die Änderung der Eigenschaften der Indikatorelemente und damit die Farbänderung visuell wahrnehmbar, lässt sich durch lnaugenscheinnahme des Reinigungsindikators nach Beendigung des Reinigungsvorgangs mit geringem Aufwand eine Einschätzung der Reinigungswirkung erzielen.

Erfindungsgemäß umfasst die Änderung der Eigenschaften der Indikatorelemente ihre Auflösung in einem im Reinigungsprozess verwendeten Reinigungsmedium. Das bedeutet beispielsweise, dass sie sich in ihren Löslichkeiten hinsichtlich des Reinigungsmediums unterscheiden. Die Auflösung eines Indikatorelementes lässt sich auf einfache Weise feststellen. Insbesondere bei einer geeigneten Anordnung der Indikatorelemente kann eine visuelle Überprüfung der Reinigungsleistung schnell erfolgen.

Die Indikatorelemente können sich durch ihre jeweiligen chemischen Vernetzungen bzw. Haftung zum Träger unterscheiden. Auf diese Weise können unterschiedliche Änderungen der Beschaffenheit der Indikatorelemente, beispielsweise ihre Löslichkeitseigenschaften hinsichtlich des Reinigungsmediums, realisiert werden. Weiterhin können sich die Indikatorelemente auch In ihrer Auftragsdicke oder ihren räumlichen Ausmaßen voneinander unterscheiden. Insbesondere können Indikatorelemente in Punkt- oder Streifenform auf dem Träger aufgebracht sein.

Eine differenzierte Aussage über die Reinigungswirkung lässt sich insbesondere dann gewinnen, wenn die Indikatorelemente nacheinander und/oder übereinander gedruckt auf dem Träger angebracht sind. Eine seitliche Anordnung der Indikatorelemente auf dem Träger erlaubt eine Gesamtübersicht über die Eigenschaften der Indikatorelemente und damit ihre Anzeige und somit eine schnelle Prüfung der Reinigungswirkung. Sind die Indikatorelemente übereinander auf dem Träger angebracht und bilden auf diese Weise gewissermaßen Bündel, lassen sich die Aussagen über die Reinigungsleistung in alternativer Weise erreichen. Sind die Indikatorelemente beispielsweise mit fallender Reinigungsleistung übereinander angebracht und lösen sich diese bei Erreichen der Reinigungsleistung im Reinigungsmedium auf, so lässt sich anhand der Indikatorelemente, die nach dem Reinigungsprozess noch auf dem Träger vorhanden sind, die Reinigungswirkung bestimmen. Die Schichtdicke der noch verbleibenden Indikatorelemente kann gewis sermaßen als Maß für die erzielte Reinigungsleistung herangezogen werden. Dabei können die Konfigurationen, in denen die Indikatorelemente nebeneinander bzw. übereinander auf dem Träger angebracht sind, in vorteilhafter Weise kombiniert werden.

Darüberhinaus erweist es sich als vorteilhaft, die Indikatorelemente in unterschiedlicher Reihenfolge übereinander auf dem Träger anzubringen. Jedes dieser Bündel von Indikatorelementen repräsentiert eine andere Anforderung an die Reinigungswirkung und liefert dementsprechend eine andere Anzeige. Entspricht beispielsweise in einem Bündel das zuoberst aufgedruckte Indikatorelement der höchsten Anforderung an die Reinigungsleistung, so muss diese Reinigungsleistung erreicht werden, damit es sich auflöst, und sich die darunterliegenden Indikatorelemente auflösen können. Auf diese Weise kann bei vollständiger Auflösung des Bündels zuverlässig festgestellt werden, dass die entsprechende Reinigungsleistung erreicht wurde. Durch mehrere solcher Bündel auf einem gemeinsamen Träger, in denen die Indikatorelemente jeweils in unterschiedlicher Reihenfolge aufgedruckt sind, kann in zuverlässiger Weise auf die im Reinigungsprozess aufgetretenen Reinigungswirkungen geschlossen werden. Haben sich in allen Bündeln alle Indikatorelemente gelöst, sind mit großer Sicherheit alle gewünschten Reinigungsleistungen bzw. Reinigungswirkungen erreicht worden.

Die Indikatorelemente sind erfindungsgemäß auf einem gemeinsamen Träger aufgebracht. Das Aufbringen kann durch ein Standarddruckverfahren bewerkstelligt werden. Insbesondere eignen sich dafür Offsetdruck, Flexodruck oder Siebdruck. Durch die Herstellung im Druckverfahren können mehrere unterschiedliche Indilcatorelemente nacheinander auf den Träger aufgedruckt werden, wodurch eine preisgünstige und einfach reproduzierbare Herstellung des Reinigungsindikators ermöglicht wird. Der Träger besteht vorteilhafterweise aus wasserfestem Material, insbesondere geeignet dafür sind Papier-Kunststofflaminat, Metallfolie oder Metallfolienlaminat.

Der Reinigungsindikator umfasst vorzugsweise zwischen zwei und zehn, und insbesondere fünf Indikatorelemente. Die Anzahl der Indikatorelemente richtet sich nach dem Bedarf und der Auflösung beziehungsweise der Genauigkeit, mit der der Reinigungsvorgang und die stattgefundene Reinigungsleistung bestimmt werden sollen.

In Bezug auf den Prüfkörperwird die oben genannte Aufgabe gelöst durch die Merkmale des Anspruchs 10

Prüfkörper oder Prüfkörpersysteme können vorteilhaft zur Überprüfung der Reinigungsleistung eines Reinigungsprozesses verwendet werden. Bei derartigen Prüfkörpersystemen wird die vergleichsweise unzugängliche Innenoberfläche komple.xer Instrumente durch ein geeignetes Modell nachgebildet, über das der Erfolg der Reinigungsmaßnahme in analoger Weise auch für vergleichsweise komplexe Instrumente überprüft werden kann. Falls bei der Verwendung eines derartigen Systems der im Prüfkörper angebrachte Reinigungsindikator eine erfolgte Reinigungswirkung anzeigt, kann davon ausgegangen werden, dass - gegebenenfalls unter Berücksichtigung eines geeignet gewählten Sicherheitsaufschlags - in den zu reinigenden Instrumenten auch an deren unzugänglichsten Stellen ihrer Innenoberfläche ein ausreichender Kontakt mit dem Reinigungsmedium erfolgt sein muss.

Erfingdungsgemäß ist der Prüfkörper als Hohlkörper ausgebildet, wobei das Material des Prüfkörpers das Detektorvolumen und das Hindernis umrandet. Der Reinigungsindikator befindet sich im Detektorvolumen innerhalb der Ummantelung. Dabei ist erfindungsgemäß der Detektorraum als Schlauchstück ausgebildet. Ein Reinigungsindikator, der auch als Detektor betrachtet werden kann, ist eingangsseitig mit einem in seiner Länge geeignet gewählten, an seinem Einlassende für Reinigungsmedium offen gehaltenen Schlauch verbunden. Der Schlauch oder Hohlkörper modelliert dabei das Verhalten ähnlich gestalteter Innenoberflächen in den zur Reinigung vorgesehenen Instrumenten. Vorteilhafterweise weist der Prüfkörper mehrere Schlauchabschnitte mit unterschiedlichem Querschnitt und/oder Volumen auf. Dies ist dadurch motiviert, dass bei üblichen Prüfkörpern eine hohe Nachweisempfindlichkeit nur mit entsprechend groß dimensioniertem Hindernis und somit gerade auf Kosten der Kompaktheit erreichbar ist. Für eine kompakte Bauweise sollte daher die Zuführung des Reinigungsmediums zum Detektorvolumen mehrstufig ausgestattet werden. Jede dieser Stufen weist einen unterschiedlichen Querschnitt auf. Dabei verändern sich je nach Querschnitt Druck und Störmungsgeschwindigkeit. Im Falle der Erhöhung des Querschnitts wird damit automatisch die Strömungsgeschwindigkeit reduziert und damit die mechanische Reinigungsleistung vermindert. Damit können schwierigere Reinigungsbedingungen simuliert werden.

Erfindungsgemäß ist der hohle Prüfkörper mit einem durchströmbaren Schüttbett ausgestattet, wie diese in der Säulenchromatographie häufig verwendet werden, kann dabei die Schüttung auch mit einem Reinigungsindikator belegt werden. Die Detektion des Reinigungsindikators auf dem Schüttvolumen kann entweder durch Ausleeren des Schüttvolumens oder durch eine transparente Wand des Prüfkörpers beurteilt werden. Das Hindernis des Prüfkörpers ist in alternativer Ausgestaltung zumindest zweistufig, gegebenenfalls aber auch mehrstufig, ausgestaltet, wobei sich die Stufen hinsichtlich ihrer Dimensierung, also insbesondere hinsichtlich ihres jeweiligen Volumens und/oder Querschnitts, voneinander unterscheiden. Die dem Detektorvolumen unmittelbar benachbarte Stufe kann dabei dazu dienen, die Zugänglichkeit zu den jeweiligen besonders unzugänglichen Innenoberflächen des behandlungsbedürftigen Instruments zu modellieren.

Im Allgemeinen ist der Prüfkörper vorzugsweise derart ausgestaltet, dass er je nach Bedarf die Eigenschaften von soliden Instrumenten und/ oder Hohlkörpern simulieren kann. Durch Verwendung von unterschiedlichen Prüfkörpern können unterschiedliche Strömungsbedingungen, Sprühschatten und weitere charakteristische Einflüsse der Beladung und Beladungshilfen im RDG simuliert werden. Zur Beurteilung bzw. Prüfung von Reinigungsprozessen eignet sich ein Verfahren wobei ein Reinigungsindikator entweder innerhalb eines Prüfkörpers oder ohne Prüfkörper einem Reinigungsprozess ausgesetzt wird, wobei nach Beendigung des Reinigungsprozesses der Reinigungsindikator einer Auswerteeinheit zugeführt wird, die die Änderung der Eigenschaften der Indikatorelemente durch den Reinigungsprozess analysiert, und wobei aus den Analysedaten ein Kennwert für die mit dem Reinigungsprozess erzielte Reinigungswirkung abgeleitet wird.

Dieser Kennwert kann darüberhinaus an eine Ausgabeeinheit weitergeleitet werden. Die Ausgabeeinheit kann den Kennwert in einem gewünschten Format bzw. in geeigneter Darstellung ausgeben. Hierbei ist beispielsweise die Ausgabe von Zahlen und/oder audiovisuellen Signalen möglich. Die Ausgabe kann dabei auf einem Display erfolgen, einen Druckvorgang einleiten, oder mit Hilfe einer Soundausgabe erfolgen.

In einer bevorzugten Ausführungsform des Verfahrens werden die Eigenschaften der Indikatorelemente nach dem Reinigungsprozess mit ihren ursprünglichen Eigenschaften anhand von Referenzdaten verglichen. Die Referenzdaten können dabei beispielsweise in geeignet abgespeicherter und abrufbarer Form vorliegen. Weiterhin können die Referenzdaten im Vorfeld kalibriert worden sein. Insbesondere die Einbeziehung von kalibrierten Referenzdaten erlaubt eine quantitative Einstufung und Beurteilung des Reinigungsprozesses.

Das Verfahren kann beispielsweise derart durchgeführt werden, dass nach Beendigung des Reinigungsvorgangs durch die Auswerteeinheit die Anzahl der Indikatorelemente bestimmt wird, deren Eigenschaften sich beim Reinigungsprozess geändert haben. Aus dieser Anzahl lässt sich dann der Kennwert bilden, der die erzielte Reinigungswirkung kennzeichnet. Ist das Verhalten der Indikatorelemente unter gewissen Bedingungen des Reinigungsprozesses bekannt, und liegen insbesondere kalibrierte Referenzdaten vor, so lässt sich zum Beispiel die Anzahl der Indikatorelemente, deren Eigenschaften sich geändert haben, auf einen - durch die Kalibration bekannten - Bereich der Reinigungsleistung abbilden.

Die Analyse der Eigenschaften der Indikatorelemente in der Auswerteeinheit lässt sich durch computerunterstützte Methoden implementierten. Wenn die Indikatorelemente derart ausgelegt sind, dass sie sich im Reinigungsmedium lösen, kann durch einen Mustererkennungsalgorithmus , beispielsweise durch eine Identifikation von dunklen gegenüber hellen Flächen, bestimmt werden, welche Indikatorelemente sich gelöst haben. Sind die Indikatorelemente auf eine Farbänderung ausgelegt, kann eine automatische Farberkennung die Beschaffenheit der Indikatorelemente auswerten. Dabei können Farbänderungen im UV oder Infrarotbereich des Lichtes mit Sensoren, die für diese Wellenlängenbereiche ausgelegt sind, erfasst werden.

Ein oder mehrere Reinigungsindikatoren, gegebenenfalls in Kombination mit einem geeigneten Prüfkörper, zusammen mit einer Auswerteeinheit, und bei Bedarf einer Ausgabeeinheit, lassen sich als Messsystem für die Messung der Reinigungsleistung von Reinigungsprozessen verstehen.

Die mit der Erfindung erzielten Vorteile bestehen insbesondere darin, dass durch eine Mehrzahl von auf einem gemeinsamen Träger unterschiedlich auf den Reinigungsprozess reagierenden Indikatorelementen eine graduelle Aussage über die Reinigungsleistung ermöglicht wird. Dadurch kann die Reinigungsleistung individuell den Anforderungen angepasst werden und materielle, energetische und zeitliche Ressourcen können eingespart werden. Durch verschiedene Möglichkeiten der Eigenschaftsänderungen der Indikatorelemente ist der erfindungsgemäße Reinigungsindikator flexibel einsetzbar und kann individuell an die Prüfungsaufgabe angepasst werden. Ein Einsetzen des Reinigungsindikators in einen Prüfkörper gestattet zudem die Analyse der Reinigungsleistung in Hinblick auf unterschiedlich geartete Instrumente.

Ein Ausführungsbeispiel wird anhand einer Zeichnung erläutert. Darin zeigen in stark schematisierter Darstellung:
- Fig. 1: eine Draufsicht auf einen Reinigungsindikator mit einer ersten An- zeige,
- Fig. 2: eine Draufsicht des Reinigungsindikators gemäß Fig. 2 mit einer zweiten Anzeige, und
- Fig. 3: einen Querschnitt durch einen Prüfkörper, in den der Reinigungsindi- kator gemäß Fig. 1 und 2 eingesetzt ist.

Gleiche Teile sind in allen Figuren mit denselben Bezugszeichen versehen.

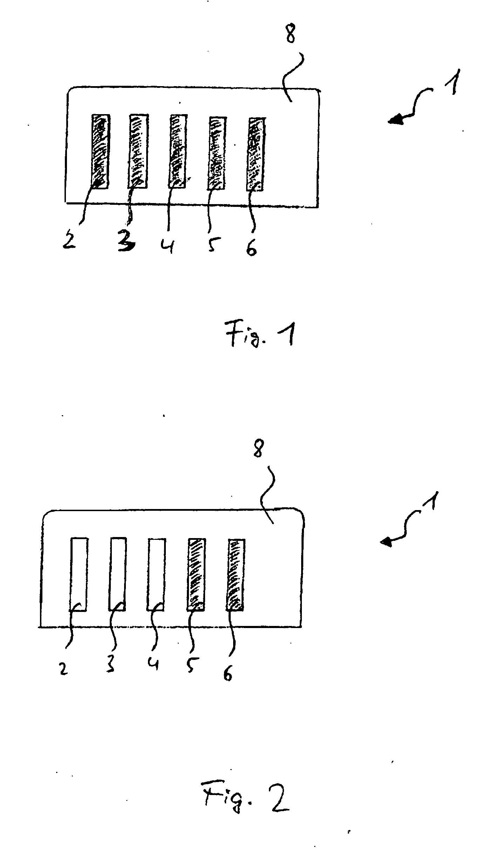

Der in Fig. 1 dargestellte Reinigungsindikator 1 ist mit fünf Indikatorelementen 2, 3, 4, 5, 6 ausgestattet, die auf einen gemeinsamen Träger 8 aufgebracht sind. Der Reinigungsindikator 1 soll hierbei seine ursprünglichen Eigenschaften - vor der Behandlung durch einen Reinigungsprozess- aufweisen und somit seine ursprüngliche Anzeige aufweisen. Die fünf Indikatorelemente 2, 3, 4, 5, 6 sind dabei derart ausgelegt, dass sie unterschiedliche Löslichkeiten in dem im Reinigungsprozess verwendeten Reinigungsmedium besitzen. Dabei ist vorgesehen, dass sich das jeweilige Indikatorelement 2, 3, 4, 5,6 vollständig auflöst, wenn die Reinigungswirkung des Reinigungsprozesses einen dem Indikatorelement 2, 3, 4, 5, 6 zugeordneten Sollwert erreicht. Die Indikatorelemente 2, 3, 4, 5 6 sind in diesem Beispiel von links nach rechts nach steigender Sensibilität in Bezug auf die Reinigungswirkung auf dem Träger 8 angeordnet. Je nach Bedarf sind auch andere Anordnungsmöglichkeiten vorstellbar und wünschenswert. Die Indikatorelemente 2, 3, 4, 5, 6 können alternativ beispielsweise in absteigender Reihenfolge oder statt nebeneinander übereinander angeordnet sein.

Eine mögliche Anzeige des Reinigungsindikators 1 nach einem Reinigungsprozess zeigt Fig. 2. Die Indikatorelemente 2, 3, 4 haben sich von dem Träger 8 gelöst. Die Indikatorelemente 5, 6 sind noch vollständig vorhanden. Somit lässt sich in diesem Beispiel schließen, das während des Reinigungsprozesses ein oder mehrere Parameter des Reinigungsprozesses nicht erreicht wurden, die zwischen dem Sollwert, der dem Indikatorelement 4 zugeordnet ist, und dem Sollwert, der dem Indikatorelement 5 zugeordnet ist, liegen. Falls die Änderungen der Beschaffenheit derart gestalteter Indikatorelemente zuvor in Testreinigungsprozessen kalibriert worden sind, lässt sich eine quantitative Angabe über die erreichte Reinigungswirkung machen.

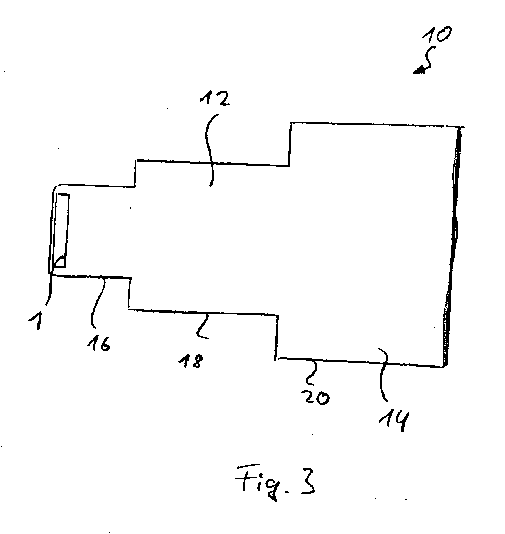

Ein Prüfkörper 10, der für die Aufnahme eines Reinigungsindikators 1 ausgelegt ist, ist in Fig. 3 dargestellt. Der Prüfkörper 10 umfasst ein Hindernis 12 für das Reinigungsmedium und eine Endmündung 14, durch die das Reinigungsmedium eintritt. Der schlauchförmige Abschnitt 16, der auch als Detektorvolumen bezeichnet wird, nimmt den Reinigungsindikator 1 auf. Der Prüfkörper 10 ist dabei aus einer Mehrzahl schlauchförmiger Abschnitte 16, 18, 20 aufgebaut, deren Querschnitt mit wachsendem Abstand vom Reinigungsindikator 1 zunimmt. Selbstverständlich können in Ergänzung einer derartigen dreistufigen Ausführung noch weitere Stufen hintereinandergeschaltet sein, oder es kann eine ein- oder zweistufige Ausführung vorgesehen sein. Durch Wahl von Anzahl und Dimension der verschiedenen Abschnitte können unterschiedliche Bedingungen des Reinigungsprozesses und unterschiedliche Eigenschaften von Instrumenten in verschiedener Ausgestaltung simuliert und getestet werden. Ein Prüfkörper kann auch zur Aufnahme von mehreren gleich oder verschieden gestalteter Reinigungsindikatoren ausgelegt sein.

### Bezugszeichenliste

- 1: Reinigungsindikator
- 2, 3, 4, 5, 6: Indikatorelemente
- 8: Träger
- 10: Prüfkörper
- 12: Hindernis
- 14: Endmündung
- 16: Detektorvolumen
- 18, 20: Abschnitt

## Patentansprüche

1. Reinigungsindikator (1) zur Prüfung von Reinigungsprozessen mit einer Mehrzahl von auf einen gemeinsamen Träger aufgebrachten Indikatorelementen (2, 3, 4, 5, 6), die in Abhängigkeit von der Reinigungswirkung eines Reinigungsprozesses ihre Eigenschaften ändern, wobei die Empfindlichkeit in Bezug auf diese Reinigungswirkung für die einzelnen Indikatorelemente (2, 3, 4, 5, 6) jeweils unterschiedlich gewählt ist, und wobei die Indikatorelemente (2, 3, 4, 5, 6) ihre Eigenschaften ändern, sobald die Reinigungswirkung einen vorgegebenen Sollwert erreicht, und wobei den einzelnen Indikatorelementen (2, 3, 4, 5, 6) jeweils unterschiedliche Sollwerte zugeordnet sind und wobei die Änderung der Eigenschaften die Auflösung des jeweiligen Indikatorelementes (2, 3, 4, 5, 6) in einem im Reinigungsprozess verwendeten Reinigungsmedium umfasst.

2. Reinigungsindikator (1) nach Anspruch 1, wobei die Änderung der Eigenschaften irreversibel ist.

3. Reinigungsindikator (1) nach Anspruch 1 oder 2, wobei die Indikatorelemente (2, 3, 4, 5, 6) unterschiedliche Haftung zum Träger aufweisen.

4. Reinigungsindikator (1) nach einem der Ansprüche 1 bis 3, wobei die Indikatorelemente (2, 3, 4, 5, 6) unterschiedliche Auftragsdicken aufweisen.

5. Reinigungsindikator (1) nach einem der Ansprüche 1 bis 4, wobei die Indikatorelemente (2, 3, 4, 5, 6) mit unterschiedlichen Eigenschaften nacheinander und/oder übereinander gedruckt auf dem Träger angeordnet sind und so eine differenzierte Aussage über die Reinigungswirkung zulassen.

6. Reinigungsindikator (1) nach einem der Ansprüche 1 bis 5, wobei die Indikatorelemente (2, 3, 4, 5, 6) mit unterschiedlichen Eigenschaften in unterschiedlicher Reihenfolge übereinander gedruckt auf dem Träger angeordnet sind, so dass unterschiedliche Anforderungen an die Reinigungswirkung entstehen.

7. Reinigungsindikator (1) nach einem der Ansprüche 1 bis 6, wobei die Indikatorelemente (2, 3, 4, 5, 6) mit einem Standarddruckverfahren, insbesondere Offsetdruck, Flexodruck oder Siebdruck, auf dem Träger (8) aufgebracht sind.

8. Reinigungsindikator (1) nach einem der Ansprüche 1 bis 7, wobei der Träger (8) aus Kunststoff, wasserfestem Papier-Kunststofflaminat, einer Metallfolie oder aus Metallfolienlaminat besteht.

9. Reinigungsindikator (1) nach einem der Ansprüche 1 bis 8 mit zwel bis zehn, insbesondere fünf Indikatorelementen (2, 3, 4, 5, 6).

10. Prüfkörper (10), der als Hohlkörper ausgestaltet ist mit einem als Schüttgut-Füllung ausgebildeten Hindernis (12) für ein im Reinigungsprozess verwendetes Reinigungsmedium und mit einem mit dem Hindernis (12) verbundenen, als Schlauchstück ausgebildetem Detektorvolumen (16), das einen Reinigungsindikator (1) nach einem der Ansprüche 1 bis 9 aufnimmt.

11. Prüfkörper (10) nach Anspruch 10, wobei der Reinigungsindikator (1) direkt auf das Schüttgut aufgebracht wird.

12. Prüfkörper (10) nach Anspruch 10 oder 11, wobei der Prüfkörper aus transparentem Material besteht, so dass das Ergebnis der Prüfung ohne Öffnung des Prüfkörpers und Entnahme des Reinigungsindikators (1) erfasst werden kann.

13. Prüfkörper (10) nach einem der Ansprüche 10 bis 12, der mehrere Volumenabschnitte mit unterschiedlichem Querschnitt und/oder Volumen aufweist.

14. Prüfkörper (10) nach Anspruch 10, der siebförmig ausgebildet ist.

15. Prüfkörper (10) nach Anspruch 10, der Spalten enthält.

## Claims

1. A cleaning indicator (1) for testing cleaning processes, with a plurality of indicator elements (2, 3, 4, 5, 6) which are applied on a common carrier and which change their properties as a function of the cleaning action of a cleaning process, wherein the sensitivity with regard to said cleaning action is selected differently for each of the individual indicator elements (2, 3, 4, 5, 6) and wherein the indicator elements (2, 3, 4, 5, 6) change their properties as soon as the cleaning action reaches a predetermined desired value, and wherein different desired values are assigned to each of the individual indicator elements (2, 3, 4, 5, 6), and wherein the change of the properties comprises the dissolution of the respective indicator element (2, 3, 4, 5, 6) in a cleaning medium used in the cleaning process.

2. The cleansing indicator (1) of claim 1, wherein the change of the properties is irreversible.

3. The cleaning indicator (1) of claim 1 or 2, wherein the indicator elements (2, 3, 4, 5, 6) have differing adhesions to the carrier.

4. The cleaning indicator (1) of any of claims 1 to 3, wherein the indicator elements (2, 3, 4, 5, 6) have different application thicknesses.

5. The cleaning indicator (1) of any of claims 1 to 4, wherein the indicator elements (2, 3, 4, 5, 6) having different properties are arranged on the carrier in a manner printed one behind the other and/or one above the other, thus permitting a differentiated statement regarding the cleaning action.

6. The cleaning indicator (1) of any of claims 1 to 5, wherein the indicator elements (2, 3, 4, 5, 6) having different properties are arranged on the carrier in a manner printed one above the other in different sequences, so that different requirements are imposed on the cleaning action.

7. The cleaning indicator (1) of any of claims 1 to 6, wherein the indicator elements (2, 3, 4, 5, 6) are applied on the carrier (8) by a standard printing process, in particular offset printing, flexographic printing or screen printing.

8. The cleaning indicator (1) of any of claims 1 to 7, wherein the carrier (8) consists of a synthetic material, a waterproof laminate of paper and plastic, a metal foil or a metal foil laminate.

9. The cleaning indicator (1) of any of claims 1 to 8, having two to ten, in particular five, indicator elements (2, 3, 4, 5, 6).

10. A test specimen (10), configured in the form of a hollow body, having an obstacle (12), designed in the form of a bulk-material filling, for a cleaning medium used in the cleaning process and having a detector volume (16), designed in the form of a hose piece and connected with the obstacle (12), which receives a cleaning indicator (1) of any of claims 1 to 9.

11. The test specimen (10) of claim 10, wherein the cleaning indicator (1) is applied directly on the bulk material.

12. The test specimen (10) of claim 10 or 11, wherein the test specimen consists of transparent material, so that the result of the testing can be detected without opening the test specimen and removing the cleaning indicator (1).

13. The test specimen (10) of any of claims 10 to 12, having a plurality of volume sections of different cross sections and/or volumes.

14. The test specimen (10) of claim 10, designed in the form of a sieve.

15. The test specimen (10) of claim 10, containing gaps.

## Revendications

1. Indicateur de nettoyage (1) pour tester des processus de nettoyage, avec une pluralité d'éléments d'indicateur (2, 3, 4, 5, 6) qui sont appliqués sur un support commun et qui changent leurs propriétés en fonction de l'action de nettoyage d'un processus de nettoyage, dans lequel la sensibilité à dite action de nettoyage est choisie différemment pour chacun des éléments d'indicateur (2, 3, 4, 5, 6) individuels et dans lequel les éléments d'indicateur (2, 3, 4, 5, 6) changent leurs propriétés dès que l'action de nettoyage atteint une valeur exigée prédéterminée et dans lequel des valeurs exigées différentes sont affectées à chacun des éléments d'indicateur (2, 3, 4, 5, 6) individuels et dans lequel le changement des propriétés comprend la dissolution de l'élément d'indicateur (2, 3, 4, 5, 6) concerné dans un milieu de nettoyage utilisé dans le processus de nettoyage.

2. Indicateur de nettoyage (1) selon la revendication 1, dans lequel le changement des propriétés est irréversible.

3. Indicateur de nettoyage (1) selon la revendication 1 ou 2, dans lequel les éléments d'indicateur (2, 3, 4, 5, 6) ont des adhérences différentes au support.

4. Indicateur de nettoyage (1) selon l'une quelconque des revendications 1 à 3, dans lequel les éléments d'indicateur (2, 3, 4, 5, 6) ont des épaisseurs différentes d'application.

5. Indicateur de nettoyage (1) selon l'une quelconque des revendications 1 à 4, dans lequel les éléments d'indicateur (2, 3, 4, 5, 6) ayant des propriétés différentes sont disposés sur le support dans une manière imprimée l'un derrière l'autre et/ou l'un au-dessus de l'autre, permettant ainsi une constatation différenciée concernant l'action de nettoyage.

6. Indicateur de nettoyage (1) selon l'une quelconque des revendications 1 à 5, dans lequel les éléments d'indicateur (2, 3, 4, 5, 6) ayant des propriétés différentes sont disposés sur le support dans une manière imprimée l'un au-dessus de l'autre dans des séquences différentes, de façon que des exigences différentes sont posées à l'action de nettoyage.

7. Indicateur de nettoyage (1) selon l'une quelconque des revendications 1 à 6, dans lequel les éléments d'indicateur (2, 3, 4, 5, 6) sont appliqués sur le support (8) à l'aide d'un procédé d'impression standard, en particulier impression offset, impression flexographique ou sérigraphie.

8. Indicateur de nettoyage (1) selon l'une quelconque des revendications 1 à 7, dans lequel le support (8) se compose d'une matière synthétique, un aggloméré laminé, résistant à l'eau, de papier et de matière synthétique, une feuille métallique ou un aggloméré laminé de feuilles métalliques.

9. Indicateur de nettoyage (1) selon l'une quelconque des revendications 1 à 8, ayant deux à dix, en particulier cinq, éléments d'indicateur (2, 3, 4, 5, 6).

10. Spécimen de test (10), configuré sous la forme d'un corps creux, ayant un obstacle (12), conçu sous la forme d'un remplissage de matière en vrac, pour un milieu de nettoyage utilisé dans le processus de nettoyage et ayant un volume détecteur (16), conçu sous la forme d'une pièce de tuyau et relié à l'obstacle (12), qui reçoit l'indicateur de nettoyage (1) selon l'une quelconque des revendications 1 à 9.

11. Spécimen de test (10) selon la revendication 10, dans lequel l'indicateur de nettoyage (1) est appliqué directement sur la matière en vrac.

12. Spécimen de test (10) selon la revendication 10 ou 11, dans lequel le spécimen de test se compose d'un matériyu transparent, de manière que le résultat du test peut être détecté sans ouvrir le spécimen de test et sans enlever l'indicateur de nettoyage (1).

13. Spécimen de test (10) selon l'une quelconque des revendications 10 à 12, ayant une pluralité de sections de volume de coupes transversales et/ou de volumes différents.

14. Spécimen de test (10) selon la revendication 10, conçu sous la forme d'un crible.

15. Spécimen de test (10) selon la revendication 10, contenant des fentes.
